# EUROPEAN PATENT SPECIFICATION

(11) **EP 1 086 944 B1**
(45) Date of publication and mention of the grant of the patent: **25.05.2005**
(21) Application number: 00118241.9
(22) Date of filing: 01.09.2000
(51) Int. Cl.: C07C 229/24, C07C 229/12, C07C 227/16

(54) **Amino acid derivative composition and process for producing an amino acid derivative**
Zusammensetzung von Aminosäurederivaten und Verfahren zur Herstellung von einem Aminosäurederivat
Composition de dérivés d'amino-acide et procédé pour la préparation d'un dérivé d'amino-acide

(30) Priority: 03.09.1999 JP 24988899; 02.11.1999 JP 31223999
(43) Date of publication of application: 28.03.2001
(73) Proprietor: Nippon Shokubai Co., Ltd., Osaka-shi, Osaka 541-0043 (JP)
(72) Inventor: Sumida, Yasutaka, Neyagawa-shi, Osaka 572-0051 (JP); Kitajima, Mitsuhiro, Suita-shi, Osaka 564-0035 (JP); Ina, Tomomi, Osaka-shi, Osaka 533-0005 (JP)
(74) Representative: Brehm, Hans-Peter

(56) References cited:
- EP-A- 0 984 101
- WO-A-00/26398
- WO-A-97/45396
- WO-A-99/25919
- PATENT ABSTRACTS OF JAPAN vol. 1996, no. 12, 26 December 1996 (1996-12-26) & JP 08 208569 A (NIPPON SHOKUBAI CO LTD), 13 August 1996 (1996-08-13)
- PATENT ABSTRACTS OF JAPAN vol. 018, no. 298 (P-1749), 7 June 1994 (1994-06-07) & JP 06 059422 A (FUJI PHOTO FILM CO LTD), 4 March 1994 (1994-03-04)
- PASCAL M L: "N 89.-SYNTHESE DE MORPHOLONES-2, DES HYDROXYAMINOACIDES CORRESPONDANT ET DE LEURS CHELATES CUIVRIQUES, A PARTIR DES EPOXYDES-1,2 ET DES SELS DE SODIUM D'ALPHA-AMINOACIDES" BULLETIN DE LA SOCIETE CHIMIQUE DE FRANCE,SOCIETE FRANCAISE DE CHIMIE. PARIS,FR, 1960, pages 435-442, XP000960955 ISSN: 0037-8968
- CHEMICAL ABSTRACTS, vol. 108, no. 5, 1 February 1988 (1988-02-01) Columbus, Ohio, US; abstract no. 37203t, ABRAMOVSKAYA ET AL.: "Synthesis and study of hydroxyethyl derivatives of monoamine-type complexing agents." page 573; column 2; XP002159600 & PROBL. KHIM. KOMPLEKSONOV, 1985, pages 108-115,

## Description

### FIELD OF THE INVENTION

The present invention relates to an L-aspartic acid derivative, to an L-aspartic acid derivative composition, further to a process for producing an L-aspartic acid derivative, and an L-aspartic acid derivative composition, and further to the use of this L-aspartic acid derivative and this L-aspartic acid derivative composition as a chelating agent. Further, the present invention relates to a process for producing N-bishydroxyalkyl-amino acid derivatives.

### PRIOR ART

Chelating agents can sequester metal ions by forming two or more coordinate bonds and are therefore used for eliminating harmful influences exerted by the presence of metal ions in various fields, such as in the fields of detergents, textiles, paper and pulp, metal surface treatment and photography.

For instance, in the field of detergents, chelating agents are used to remove metal ions, such as calcium and magnesium ions; this allows the use of hard water for the intended purpose. In the field of textiles, paper and pulp, chelating agents sequester metal ions, thus hindering those metal ions to decompose hydrogen peroxide and other bleaching agents.

In this respect, a typical chelating agent, is ethylenediaminetetraacetic acid (EDTA), which is inexpensive and provides high chelating power.

Following intended use, these chelating agents are contained in household waste water and in industrial waste water. Therefore, these chelating agents may have unfavourable influences on the environment and ecological system, if they are hardly biodegradable. Generally, the term "biodegradability" means that a substance can be decomposed spontaneously by microorganism or products thereof in nature and the decomposition products do not have any longer harmful influences on the environment and ecosystem.

In recent years, the regulations against environmental pollution by chemical substances have become more and more strict. According to the current situation, those chelating agents having poor or lacking biodegradability face problems of approval. Therefore, various investigations search for chelating agents having improved biodegradability.

Japanese Kokai Publication Hei-08-208569 is related to a cleaner composition which may comprise as essential component a diethanolamine derivative represented by the following general formula

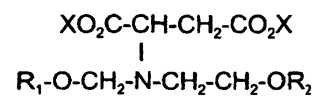

This diethanolamine derivative is produced by reacting diethanolamine with maleic acid and/or a salt thereof in the presence of an alkaline earth metal. The chelating effect is due to carboxyl groups located around the nitrogen atom and to the ether group-derived oxygen atom, which serve as central atoms. This diethanol derivative provides an outstanding high level of biodegradability not reached by other conventional chelating agents.

Japanese Kohyo Publication Hei-10-502632 discloses a process for producing salts of [S,S]-ethylenediamine-N,N'-disuccinic acid (ss-EDDS) which comprises the step of reacting L-aspartic acid with a 1,2-dihaloethane in a basic aqueous solution in which the amount of 1,2-dihaloethane is stoichiometrically insufficient throughout the reaction period so that less than 60 mole percent of the initial charge of L-aspartic acid may react. It has been found that the sodium salt of the obtained ss-EDDS is useful as a biodegradable chelating agent. Laid-open International Patent Specification WO 97/45396 discloses N-bis- or N-tris[(1,2-dicarboxy-ethoxy)-ethyl]-amine derivatives for example compounds such as
- N-bis-[(1,2-dicarboxy-ethoxy)-ethyl]-amine (BCEEA),
- N-tris-[(1,2-dicarboxy-ethoxy)-ethyl]-amine (TCEEA), and
- N-bis-[(1,2-dicarboxy-ethoxy)-ethyl]-aspartic acid (BCEEAA),
as well as the alkali meta and earth-alkali metal salts of the said compounds, preferably Na⁺, K⁺, Ca²⁺ and Mg²⁺ salts as a biodegradable chelating agent. This amine derivative may be obtained by reacting di- or triethanolamine with maleic acid in the presence of an alkali metal salt or alkaline earth metal salt, using a lanthanide compound, a lanthanide compound mixture or an alkaline earth metal compound as a catalyst. The obtained compounds comprise two or more asymmetric carbon atoms, but for the time being, the configurations thereof have not been specified.

Japanese Kokai Publication Hei-06-59422 discloses a treatment composition for silver halide-containing photosensitive materials which contains an N-bishydroxyethylaspartic acid salt or the like as a biodegradable chelating agent.

Problemy Khimii Kompleksonov (1985) (in Russian), pages 108-115, discloses a process for producing N-bishydroxyethylaspartic acids. This process comprises several steps including esterifying aspartic acid in a methanol/hydrochloric acid solution to provide dimethyl aspartate, further the step of subjecting the obtained dimethyl aspartate to an addition reaction with ethylene oxide, and the subsequent step of hydrolyzing the addition product with sodium hydroxide or the like. This process involves a number of steps and provides a high amount of byproducts, therefore the yield is poor. Further, the production costs are high and it is difficult to improve the yield.

The document EP-A-0 984 101 is related to a sizing composition usable for the neutral sizing of paper or board. This sizing composition may comprise an organic complexing agent selected from a group of compounds including
- N-bis-[(1,2-dicarboxy-ethoxy)-ethyl]-amine (BCEEA),
- N-bis-[(1,2-dicarboxy-ethoxy)-ethyl]-aspartic acid (BCEEAA), or
- N-tris-[(1,2-dicarboxy-ethoxy)-ethyl]-amine (TCEEA),
or a salt thereof or a mixture thereof.

The document WO-A-99/25919 is related to a process for the bleaching of chemical pulp, wherein the pulp is delignified and/or bleached with chlorine dioxide or with a combination of chlorine dioxide and a per-compound, and additionally the pulp is chelated in order to bind heavy metals, such as Fe, Mn and/or Cu, to a chelate complex. The chelating agent is selected from a group comprising N-bis-[(1,2-dicarboxy-ethoxy)-ethyl]-amine (BCEEA), N-bis-[(1,2-dicarboxy-ethoxy)-ethyl]-aspartic acid (BCEEAA) and N-tris-[(1,2-dicarboxy-ethoxy)-ethyl]-amine (TCEEA) and the alkali metal and earth-alkali metal salts of these. The chelating treatment is carried out simultaneously by combining the chelating agent with the pulp in the same bleaching stage as chlorine dioxide.

A scientific essay of Maurice L. Pascal publishes in Bulletin de la Societé Chimique de France, 1960, pages 435 - 442 (1959) is related to the synthesis of certain morpholone-2-compounds. An intermediate product is N,N-bis(hydroxy-2-ethyl)-α-aminopropionic acid, which forms two different stereoisomers d(-) and d(+), which have been prepared and characterised. Further, each a copper chelate complex of this different stereoisomers has been prepared. Further, this document discloses a process for producing (S)-N, N-bishydroxyethylglutamic acid of the formula HOOC-CH₂-CH₂-C*H(COOH)-N(CH₂-CH₂-OH)₂ by adding an aqueous solution of sodium hydroxide to (S)-glutamic acid [I(+)-glutamic acid] and reacting the sodium salt of (S)-glutamic acid thus formed with ethylene oxide (see page 441, Experiment XI).

The document WO-A-00/26398 published on May 11, 2000 is related to a method of producing aspartic acid derivatives of general formula (I) and their mixtures 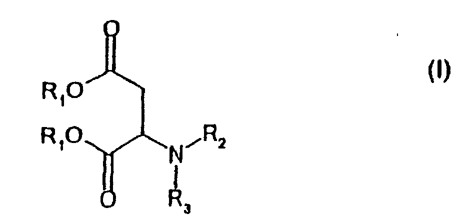 wherein
R₁ may represent H, Na, K, NH₄, Ca, Mg, Li or Fe;
R₂ may represent one of the following groups: 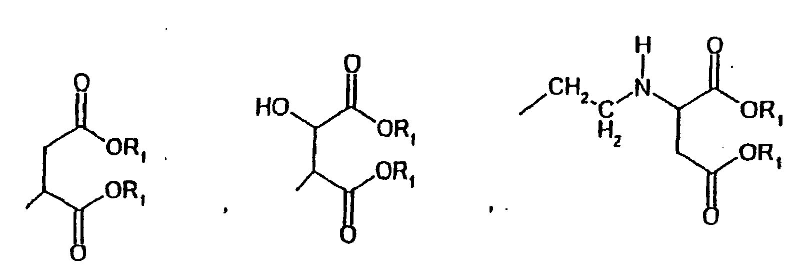 -CH₂-COOR₁, -CH₂-CH₂-COOR₁, -CH₂-CH₂-OH, -CH₂CHOH-CH₃ or -CH₂-CHOH-CH₂OH; and
R₃ may represent H, -CH₂-COOR₁, -CH₂-CH₂-COOR₁, -CH₂-CH₂-OH, -CH₂-CHOH-CH₃ or -CH₂-CHOH-CH₂OH.

The method contains a step of converting fumaric acid di(ammonium salt) by means of enzymes, especially by means of aspartase into mono- or di-ammonium salts of aspartic acid. The aspartic acid is obtained to the major extent, preferably in an extent larger than 90 % in the S form. The thus obtained aspartic acid derivatives and their mixtures form biologically degradabilable complexing agents.

Chemical Abstracts vol. 108, no. 5, 1988, Abstract no. 37203 t is related to synthesis and study of hydroxyethyl derivatives of monoamine-type complexing agents. Accordingly, HOCH₂CH₂N[CH(CO₂H)CH₂CO₂H]₂, and the mono- and bis(hydroxyethyl) derivatives of aspartic and glutamic acids were prepared (as complexions) by esterifying the acids with MeOH-HCl, treating with oxirane, and hydrolyzing with NaOH or LiOH.

### SUMMARY OF THE INVENTION

Starting from this prior art, it is the object of the present invention to provide a further composition or compound having sufficient chelating activity and remarkably high biodegradability. According to a further related object, the present invention states a process for producing said composition or compound. According to a further related object, the present invention provides a simplified process for producing in improved yields and reduced amount of by products N-bishydroxyalkyl-amino acid derivatives having high chelating activity and improved biodegradability.

In order to solve the fore-mentioned object(s), the present invention provides an L-aspartic acid derivative represented by the following general formula (2): 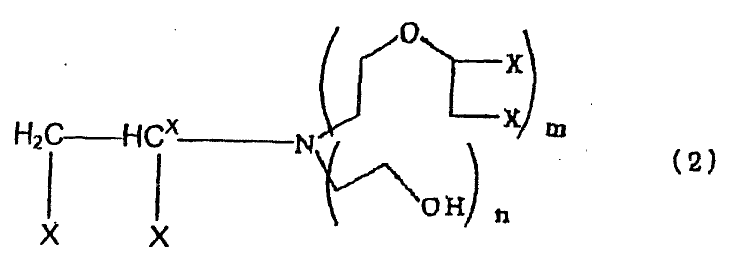 wherein:
C^{x} indicates an asymmetric carbon atom being in the S configuration;
X represents COOM, wherein all the M are the same or different and each represents a hydrogen atom, an alkali metal atom, an alkaline earth metal atom or an ammonium group;
m represents an integer of 1 or 2; and
n represents an integer of 0 or 1 with the further proviso that m + n is equal to 2.

According to a further aspect, the present invention provides an L-aspartic acid derivative composition comprising an L-aspartic acid derivative (A) and an L-aspartic acid derivative (B)
wherein said L-aspartic acid derivative (A) being represented by the following general formula (3): 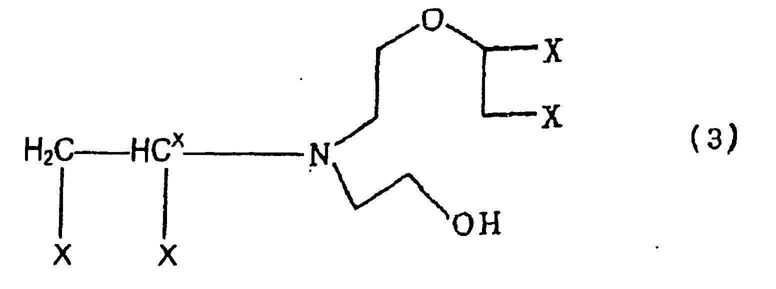 wherein:
C^{x} indicates an asymmetric carbon atom being in the S configuration;
X represents COOM, wherein all the M are the same or different and each represents a hydrogen atom, an alkali metal atom, an alkaline earth metal atom or an ammonium group; and
wherein said L-aspartic acid derivative (B) being represented by the following general formula (4): 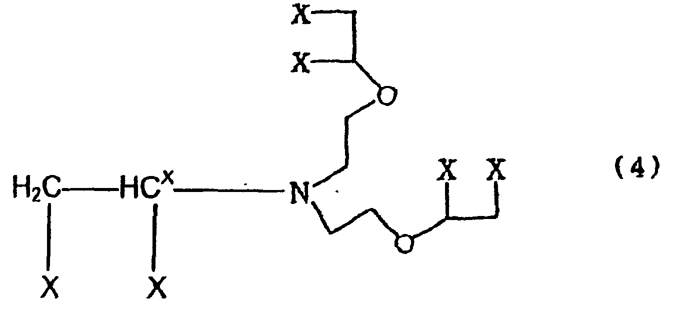 wherein:
C^{x} indicates an asymmetric carbon atom in the S configuration;
X represents a hydrogen atom, an alkali metal atom, an alkaline earth metal atom or an ammonium group; and
wherein said L-aspartic acid derivative composition comprises a mole ratio of said L-aspartic acid derivative (A) to said L-aspartic acid derivative (B) in the range of from 1 : 99 to 99 : 1.

According to a further aspect, the present invention provides a process for producing the L-aspartic acid derivative according to the fore-mentioned general formula (2), which process comprises the steps of:
- subjecting L-aspartic acid to an addition reaction with ethylene oxide in order to obtain a L-aspartic acid/ethylene oxide adduct; and
- reacting said L-aspartic acid-ethylene oxide adduct with maleic acid in the presence of a rare earth-based catalyst.

According to a further aspect, the present invention provides a process for producing the fore-mentioned L-aspartic acid derivative composition which process comprises the steps of:
- subjecting L-aspartic acid to an addition reaction with ethylene oxide in order to obtain a L-aspartic acid/ethylene oxide adduct; and
- reacting said L-aspartic acid-ethylene oxide adduct with maleic acid in the presence of a rare earth-based catalyst.

According to a still further aspect, the present invention provides a process for producing N-bishydroxyalkyl-amino acid derivatives represented by the following general formula (5): 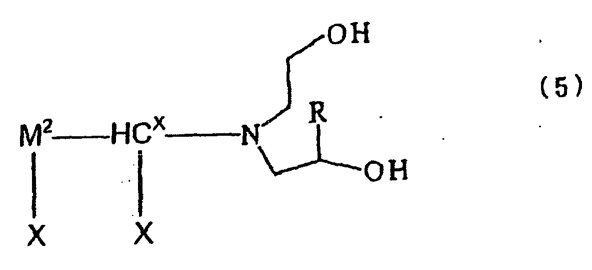 wherein:
C^{x} indicates an asymmetric carbon atom in the S configuration;
R represents a hydrogen atom; M² represents -CH₂- group;
X represents COOM, wherein all the M are the same or different and each represents a hydrogen atom, an alkali metal atom, an alkaline earth metal atom or an ammonium group;
which comprises the step of effecting an addition reaction of an alkylene oxide to an amino acid salt in an aqueous solution.

A preferred embodiment of the above-mentioned process for preparing N-bishydroxyalkyl-amino acid derivatives
is a process wherein:
R represents a hydrogen atom; and
comprising the step of effecting an addition reaction of ethylene oxide to an aspartic acid salt in an aqueous solution.

### BRIEF DESCRIPTION OF THE DRAWING

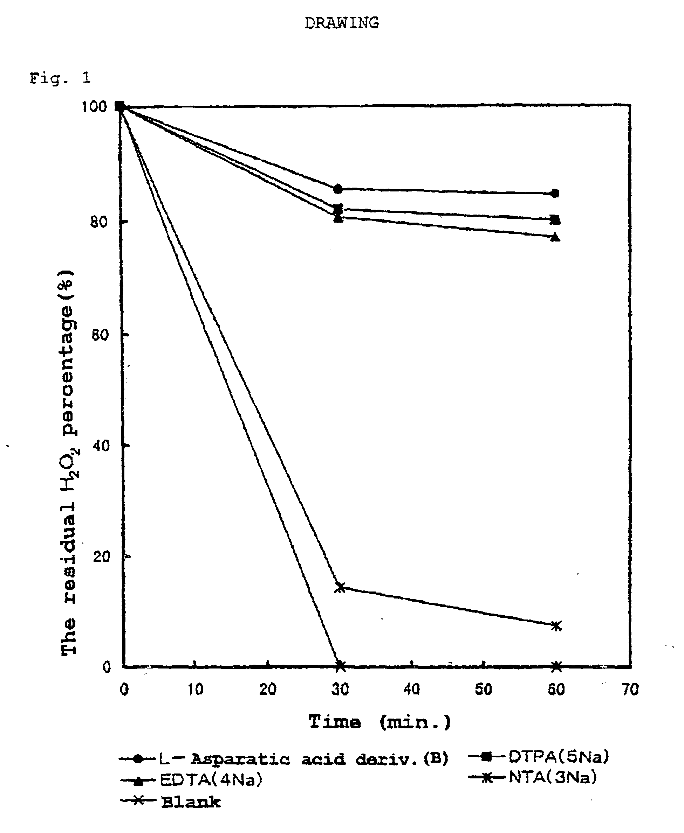

Fig. 1 is a graph representing the time-dependant decrease of residual H₂O₂ in a test demonstrating the Ca²⁺ion trapping capability of several tested compounds.

### DETAILED DESCRIPTION OF THE INVENTION

In the following, the present invention is described in more detail.

The present invention is related to an L-aspartic acid derivative represented by the following general formula (2): 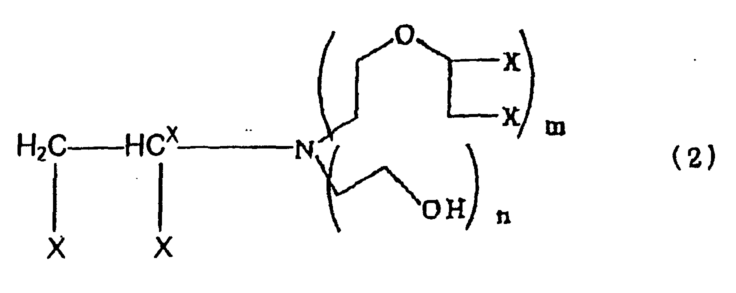 wherein:
C^{x} indicates an asymmetric carbon atom being in the S configuration;
X represents COOM, wherein all the M are the same or different and each represents a hydrogen atom, an alkali metal atom, an alkaline earth metal atom or an ammonium group;
m represents an integer of 1 or 2; and
n represents an integer of 0 or 1 with the further proviso that m + n is equal to 2.

In case, "m" equals 1 and "n" equals 1 an L-aspartic acid derivative (A) is obtained having a structure according to the following general formula (3):

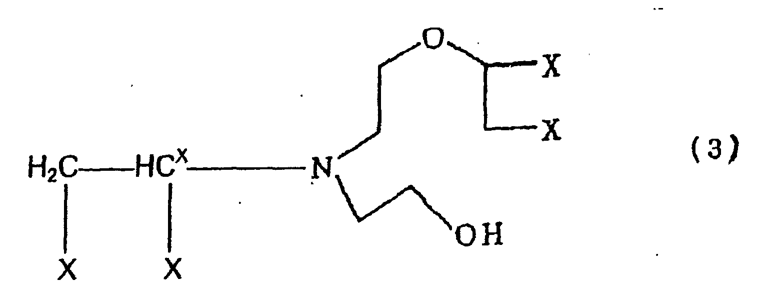

In case, "m" equals 2 and "n" equals O, an L-aspartic acid derivative (B) is obtained having a structure according to the following general formula (4):

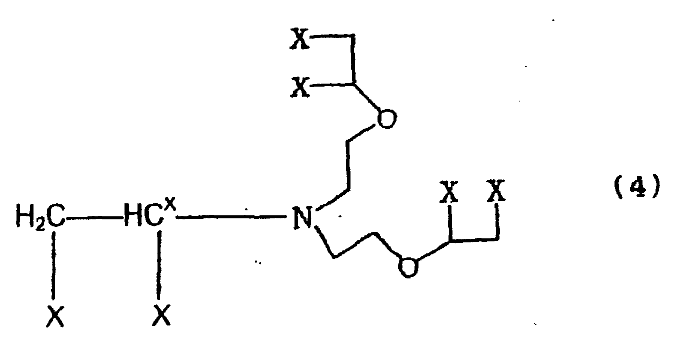

Evidently, all the L-aspartic acid derivatives according to the present invention comprise a chemical structure wherein two or more carboxyl or substituted carboxyl groups and the nitrogen atom and ether group-derived oxygen atom are present within one and the same molecule.

The alkali metal atom mentioned above is not particularly restricted but includes lithium, sodium, potassium, rubidium, cesium and francium. Among them, sodium and potassium are preferred, however, because of their ready availability and inexpensiveness. The above alkaline earth metal atom is not particularly restricted but includes beryllium, magnesium, calcium, strontium, barium and radium. Magnesium and calcium are preferred, however, because of their ready availability and inexpensiveness.

Based on their characteristic chemical structure, the above L-aspartic acid derivatives can serve as at least tetraentate ligands to metal ions and, therefore, the L-aspartic acid derivative composition of the present invention show good chelating activity. When it contains the derivative (B) in which "n" is 0 and "m" is 2, the L-aspartic acid derivative composition of the invention expresses still better chelating activity since the coordination number to metal ions can be further increased.

The chelating activity of the L-aspartic acid derivative composition of the invention can be measured by various methods. For example, it can be measured in the following manner.

### (1) Evaluation of chelating performance against Ca²⁺

The chelating performance against Ca²⁺ can be evaluated in terms of Ca²⁺ trapping ability by the calcium ion-selective electrode method mentioned below.
[1] 2 ml of 4 M KCl is added to 100 ml of 10⁻³ M CaCl₂ solution.
[2] 10 mg of the sample is added to the above solution, and the solution is adjusted to pH 10 with 0.1 M KOH (or 0.1 M HCl) and maintained at 25 °C.
[3] The Ca²⁺ concentration is measured using a calcium ion-selective electrode.
[4] The Ca²⁺ trapping capability is calculated in terms of the amount of Ca²⁺ trapped based on the result of Ca²⁺ concentration measurement.

### (2) H₂O₂ stabilization test

Measurements can be made as follows. Measurement method: The stability of H₂O₂ in the alkaline region in the presence of Fe³⁺ is measured by the time-dependant decrease of the residual H₂O₂ in percentage. Measurement conditions: pH 10, 50 °C, Fe³⁺ concentration 2 ppm, initial quantity of H₂O₂ 3 g/l, sample concentration 200 ppm.

By evaluating the Ca²⁺ chelating capacity in the above manner, it is possible to estimate the chelating performance (chelating activity) in terms of calcium ion (Ca²⁺) trapping ability. The chelating performance (chelating activity) can be evaluated by the above H₂O₂ stabilization test with the heavy metal ion sequestering capability as a measure.

The biodegradability of the L-aspartic acid derivative composition of the invention can be measured by various methods. For example, it can be measured by any of the following test methods prescribed in the OECD Guideline 301 (ready degradability):
Test method 301A: DOC Die-Away test
Test method 301B: CO₂ emission test (modified Sturm test)
Test method 301C: Modified MITI test (I)
Test method 301D: Closed bottle test
Test method 301E: Modified OECD screening test
Test method 301F: Manometric respirometry test

Among these, the test method 301C: Modified MITI test (I) is preferred.

According to these test methods, biodegradability evaluation can be made by measuring such parameters as Dt (decomposition degree), BOD (biochemical oxygen demand) and CO₂ emission generally for consecutive 28 days.

The L-aspartic acid derivative composition according to the present invention has marked chelating activity as well as high biodegradability.

Why such excellent chelating activity is exhibited is no doubt associated with the positions of the carboxyl groups or substituted carboxyl groups and the number of such groups in the molecules contained in the L-aspartic acid derivative composition.

For the L-aspartic acid derivatives according to the invention to have improved chelating activity, it is necessary that they have the group: 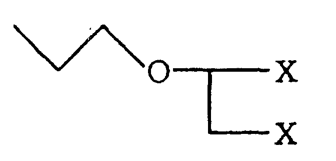 wherein X is as defined above,
and it is more preferred that they have this group in large numbers within the molecule.

For the L-aspartic acid derivatives according to the present invention to have an elevated level of biodegradability, it is mandatory that the group represented by -CHX-CH₂X and having the asymmetric carbon atom as indicated by the asterix in this group be in S configuration.

The reasons why the L-aspartic acid derivative composition according to the present invention shows biodegradability are not fully clear. However, the following reasons, for instance, are plausible:
(1) That compounds having a tertiary amine structure, when an atom or group of atoms readily undergoing biodegradation is bonded to the tertiary amine-constituting nitrogen atom, are presumably susceptible to biodegradation; (2) that the occurrence of a hydroxy group leads to ready biodegradability;
(3) that when the group -CHX-CH₂X and the asymmetric carbon atom in this group is in S configuration, the possibility of the asymmetric carbon atom in S configuration undergoing biodegradation is essentially higher as compared with the asymmetric carbon atom in R configuration since the asymmetric carbon atoms in naturally occurring compounds have in principle the S configuration; and (4) that since biodegradation of a compound seemingly proceeds in the manner of chain reaction once one atom or atomic group forming the compound has undergone biodegradation, compounds having at least one readily biodegradable atom or atomic group are considered to be readily biodegradable.

In other words, it is presumable that the above L-aspartic acid derivatives acquire an elevated level of biodegradability owing to its tertiary amine structure itself and, in addition, that since when, for example, "m" in general formula (2) is 1 and "n" is 1, they have a hydroxy group and, when the group represented by -CHX-CH₂X and the asymmetric carbon atom in this group is in S configuration, they have an asymmetric carbon atom in S configuration with the asymmetric carbon atom in S configuration being bonded to the nitrogen atom in their tertiary amine structure, they, which have such a hydroxy group and such an asymmetric carbon atom that are readily biodegradable, may effectively be biodegraded. It is further considered that when such hydroxyl group and asymmetric carbon atom are biodegraded, the derivatives are biodegraded in a chain reaction manner, manifesting a high level of biodegradability.

The term "L-aspartic acid derivative" as used herein does not mean a compound obtained by using L-aspartic acid as the starting material but only means that it is a compound having the basic skeleton of L-aspartic acid within the molecule.

One of the characteristic features from the chemical structure viewpoint of the L-aspartic acid derivative of the present invention is that at least four carboxyl or substituted carboxyl groups, a nitrogen atom and an ether group-derived oxygen atom or atoms are present simultaneously in one and the same molecule.

Owing to the chemical structure feature, the L-aspartic acid derivative of the invention can serve as an at least hexadentate ligand against metal ions and therefore can exhibit good chelating activity. Among the L-aspartic acid derivatives covered by the present invention, those in which "n" is 0 and "m" is 2 may have a still increased coordination number against metal ions and therefore can exhibit still better chelating activity.

Among the L-aspartic acid derivatives according to the present invention, the compound in which "n" is 1, "m' is 1 and M is hydrogen and in which the asymmetric carbon atom carrying the asterix * has an unspecified configuration is the compound described as BCEEAA in Laid-open International Patent Specification WO 97/45395. However, while said BCEEAA has a total of three asymmetric carbon atoms in its molecule, the configuration of any of the three symmetric carbon atoms has not been specified, hence said BCEEAA should be construed as being a compound different from the L-aspartic acid derivative of the present invention in which the configuration for one asymmetric carbon atom has been specified. Further, since it is one of the structural features of the L-aspartic acid derivative of the present invention that one asymmetric carbon atom of which the configuration is specified is bonded to the tertiary amine-constituting nitrogen atom, the BCEEAA and the L-aspartic acid derivative of the present invention should be construed as being compounds different from each other. Furthermore, the L-aspartic acid derivative of the present invention differs in the effect as a chelating agent and, in particular, in biodegradability from BCEEAA and, therefore, the present invention is distinct from the invention described in the above-cited laid-open international patent specification.

The L-aspartic acid derivative of the present invention has excellent chelating activity as well as high biodegradability. Here, it may be pointed out that the L-aspartic acid derivative of the invention has a plurality of groups having a chelating activity-enhancing effect. Further, the L-aspartic acid derivative of the invention has an asymmetric carbon atom showing an S configuration and, in its tertiary amine structure, the above asymmetric carbon atom in S configuration is bonded to the nitrogen atom and, when, for example, "m" in general formula (2) is 2 and "n" is 0, the derivative has a hydroxy group and, owing to the presence of such asymmetric carbon atom and hydroxy group, which are readily undergo biodegradation, it is probably biodegraded effectively. It is also conceivable that once asymmetric carbon and hydroxy group have been biodegraded, the biodegradation can proceed in a chain reaction manner, resulting in high biodegradability.

The L-aspartic acid derivative of the invention, which is represented by the general formula (2), when "m" is 1 and "n" is 1, for instance, becomes an L-aspartic acid derivative (A) represented by the general formula (3) and, when "m" is 2 and "n" is 0, it becomes an L-aspartic acid derivative (A) represented by the general formula (4). Therefore, the L-aspartic acid derivative of the invention includes two subclasses, namely the above L-aspartic acid derivative (A) and L-aspartic acid derivative (B).

One of the features of the L-aspartic acid derivative of the invention lies in the fact that the asymmetric carbon atom carrying the asterix * in the above stated general formula (2) is in S configuration. While, in the general formula (2), there are three asymmetric carbon atoms, the configuration of the other asymmetric carbon atoms than the one marked with * need not be specific. The S configuration and R configuration may occur together without any restriction with regard to the ratio therebetween. Therefore, the L-aspartic acid derivative of the present invention is a compound in which the configuration of only the one asymmetric carbon atom marked with * out of the three asymmetric carbon atoms occurring in the molecule is specified as S.

When the L-aspartic acid derivative of the invention is produced by the process for producing L-aspartic acid derivatives according to the present invention, which is mentioned later herein, the configuration of the starting material L-aspartic acid is retained, hence the L-aspartic acid-derived configuration is retained as it is as the S configuration of the asymmetric carbon atom marked with * in the structural formula given as general formula (2).

In the present specification, the step of effecting an addition reaction of ethylene oxide to an L-aspartic acid derivative is referred to as "ethylene oxide addition step" and the step of reacting the L-aspartic acid derivative-ethylene oxide adduct with maleic acid in the presence of a rare earth-based catalyst is referred to as "maleic acid addition step".

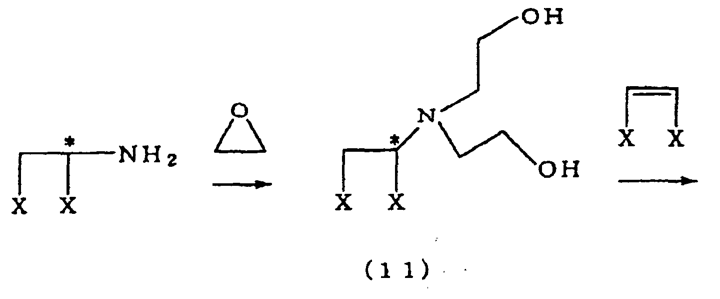

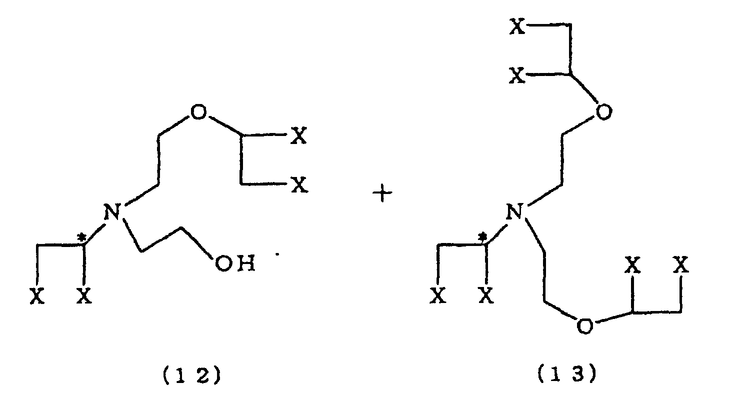

In the above reaction formula, X is defined as stated above.

As the above stated ethylene oxide adduct, there may generally be mentioned an ethylene oxide adduct (general formula (11) given above) resulting from complete substitution of the two hydrogen atoms of the amino group in L-aspartic acid by ethylene oxide. The thus-formed ethylene oxide adduct represented by the general formula (11) is generally subjected to maleic acid addition reaction to give the maleic acid adduct. As the thus-obtainable maleic acid adduct, there may be mentioned the maleic acid adduct 1 (general formula (12) given above) resulting from addition of one molecule of maleic acid and the maleic acid adduct 2 (general formula (13) given above) resulting from addition of two molecules of maleic acid.

The above L-aspartic acid derivative production process also constitutes an aspect of the present invention. According to the process of the invention, there is no possibility at all of formation of the compound represented by the following general formula (10): 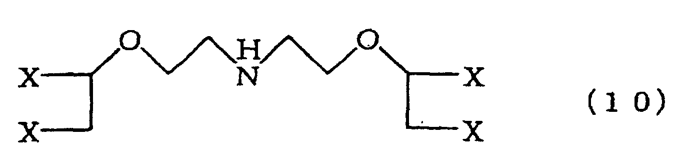 wherein X has the above stated meaning in this respect the L-aspartic acid derivative production process of the present invention is quite different from the production process disclosed in Laid-open International Patent Specification WO 97/45396. The compound represented by the above general formula (10) is inferior in chelating activity and in biodegradability and a compound is quite different from the L-aspartic acid derivative of the present invention.

In the above stated maleic acid addition step, maleic acid or maleic anhydride may be used. For carrying out the reaction efficiently, the use of maleic anhydride as the raw material is preferred.

The rare earth-based catalyst to be used in the above unsaturated carboxylic acid addition reaction is not particularly restricted but generally has the form of oxide, hydroxide, chloride, nitrate, sulfate, oxalate or the like. As the rare earth element, there may be mentioned lanthanoid elements such as lanthanum, cerium, praseodymium and neodymium; scandium; yttrium and so forth. Among them, lanthanum is relatively inexpensive and readily available. Lanthanum oxide is thus preferred.

In the above stated ethylene oxide addition step the mole ratio between L-aspartic acid and ethylene oxide, is not particularly restricted but, for both hydrogen atoms of the amino group in the L-aspartic acid to be completely substituted by the ethylene oxide, it is preferred that the ratio (number of moles of ethylene oxide) / (number of moles of L-aspartic acid) amount to 2 to 10, more preferably 2.5 to 8, still more preferably 3 to 6.

In the above stated ethylene oxide addition step, the hydroxide or carbonate of an alkali metal, the hydroxide or carbonate of an alkaline earth metal, an ammonium group-containing compound or the like is preferably used for substituting the carboxyl hydrogen atom or atoms of the L-aspartic acid.

The amount of the compound to be used for substituting the carboxyl hydrogen atom or atoms of the L-aspartic acid is not particularly restricted but, for complete substitution of the carboxyl hydrogen atom or atoms the above compound is used preferably in an amount not less than one equivalent per equivalent of the L-aspartic acid. For example, the above compound is used preferably in an amount of 1 to 3 equivalents, more preferably 1 to 2 equivalents, per equivalent of the L-aspartic acid.

In the above stated ethylene oxide addition step, the method of carrying out the reaction is not particularly restricted. Preferably, however, the reaction system is purged with an inert gas such as nitrogen gas and the reaction is carried out using a reaction medium. For controlling the reaction of the ethylene oxide, it is preferred that the ethylene oxide be added (dropwise) over a prolonged period of time.

The above reaction medium is not particularly restricted but is preferably an aqueous medium, for instance, more preferably water.

The amount of the above stated reaction medium is not particularly restricted but, generally, the reaction is carried out preferably in such a state that the L-aspartic acid salt is dissolved in the medium to give a homogeneous solution, for example at an L-aspartic acid salt concentration of about 40% by weight.

The period during which the above stated ethylene oxide is to be added (dropwise) is not particularly restricted. However, with ethylene oxide a period of 0.5 to 6 hours is preferred and 1 to 5 hours is more preferred.

The reaction temperature and reaction time in the above stated ethylene oxide addition step are not particularly restricted. With ethylene oxide the reaction temperature is preferably 15 to 80°C and the reaction time after completion of the addition (dropping) of ethylene oxide is preferably 0.5 to 5 hours. More preferably, the reaction temperature is 20 to 60 °C and the reaction time after completion of the addition (dropping) of ethylene oxide is 1 to 4 hours.

The percent conversion of the L-aspartic acid in the above stated ethylene oxide addition step is not particularly restricted but preferably amounts to not less than 60%, for instance, more preferably not less than 80%, still more preferably not less than 90%, most preferably not less than 95%.

In the above stated maleic acid addition step, the mole ratio between maleic acid and L-aspartic acid-ethylene oxide adduct is not particularly restricted but preferably the ratio (maleic acid)/(L-aspartic acid-ethylene oxide adduct) amounts to 0.5 to 4, more preferably 1 to 3.

In the above stated maleic acid addition step, it is possible to adjust the mole ratio between the maleic acid adduct 1 (derivative (A)) the maleic acid adduct 2 (derivative (B)) and in the L-aspartic acid derivative product by adjusting the mole ratio between the maleic acid and L-aspartic acid-ethylene oxide adduct.

In the above stated maleic acid addition step, the hydroxide or carbonate of an alkali metal, the hydroxide or carbonate of an alkaline earth metal, an ammonium group-containing compound or the like is preferably used for substitution of the carboxyl hydrogen atom or atoms of the maleic acid. The amount of the compound to be used for substituting the carboxyl hydrogen atom or atoms of the maleic acid is not restricted. For complete substitution of the carboxyl hydrogen atom or atoms of the maleic acid, the above stated compound is preferably used in an amount of not less than one equivalent per equivalent of the maleic acid. For example, the above stated compound is used preferably in an amount of 1 to 3 equivalents, more preferably 1 to 2 equivalents, per equivalent of the maleic acid.

The method of carrying out the reaction in the above stated maleic acid addition step is not particularly restricted. Preferably, however, the reaction is carried out while adjusting the pH of the reaction system.

The pH of the reaction system in the above stated maleic acid addition step is not particularly restricted but is preferably not lower than 8, more preferably not lower than 9, still more preferably not lower than 9.5.

When it is intended that the maleic acid adduct 1 represented by the above general formula (12) and the maleic acid adduct 2 represented by the above general formula (13) be obtained in the form of an alkali metal salt, salt exchange is effected by adding the carbonate and/or bicarbonate of the corresponding alkali metal, for instance, to thereby cause precipitation of the carbonate of the rare earth contained in the system, followed by removing the precipitate by filtration, for instance, whereby the alkali metal salt of the above maleic acid adduct 1 and the alkali metal salt of the above maleic acid adduct 2 are obtained in the form of an aqueous solution.

The composition obtained in the above stated maleic acid addition step can further be separated into the respective components, and each L-aspartic acid derivative can be isolated.

The method of the above isolation is not particularly restricted. For example, after conversion of all carboxyl groups to the acid form by acid treatment, the derivative (A) and the derivative (B) can be recovered each in the acid form by carrying out a chromatographic procedure using a column packed with a basic anion exchange resin and using an aqueous solution of formic acid, for instance, as the eluent.

When the ratio between the derivatives (A) and (B) is within a predetermined specific range the L-aspartic acid derivative composition is a very useful composition since it is not necessary to isolate the respective single derivatives. The combined product may be superior in chelating activity and biodegradability than the single isolated derivatives.

The chelating activity and biodegradability of the L-aspartic acid derivative composition of the present invention may be adjusted by adjusting the ratio between derivative (A) and derivative (B). Accordingly, when the L-aspartic acid derivative composition of the present invention is to be contained in a chelating agent, the chelating activity and biodegradability thereof can be adjusted according to the field of use thereof as a chelating agent and/or the performance characteristics required.

The ratio between the derivative (A) and the derivative (B) in the L-aspartic acid derivative composition of the present invention is generally 1:99 to 99:1. When this ratio is outside the range of 1:99 to 99:1, it becomes impossible to adjust the chelating activity and biodegradability.

This ratio is preferably 1:9 to 9:1.

When this ratio is smaller than 1:9 and the proportion of the derivative (A) becomes excessively small, the biodegradability decreases; thus it may become impossible to balance the biodegradability against the chelating activity. When this ratio is greater than 9:1 and the proportion of the derivative (A) becomes excessively large, the required chelating activity decreases; thus it may become impossible to balance the chelating activity against the biodegradability.

This ratio is more preferably 2: 8 to 8: 2, still more preferably 3:7 to 7:3, most preferably 4:6 to 6:4.

The chelating agent of the present invention may contain, in addition to the L-aspartic acid derivative and/or L-aspartic acid derivative composition of the present invention, another chelating agent and/or an additive or additives.

The chelating agent of the present invention may be utilized in a wide range of fields, for example in detergents, detergent compositions, auxiliaries for textile industry, auxiliaries for paper and pulp manufacture, metal surface treating agents and photographic developers.

The process for producing N-bishydroxyalkyl-amino acids represented by the above general formula (5) which comprises the step of effecting an addition reaction of an alkylene oxide to an amino acid salt in an aqueous solution provides the desired N-bishydroxyalkyl-amino acids in high yields while simplifying the production process and reducing the possibility of byproduct formation. Such N-bishydroxyalkyl-amino acid production process is also an aspect of the present invention.

The above stated step of effecting an addition reaction of an alkylene oxide to an amino acid salt in an aqueous solution is hereinafter referred to as "alkylene oxide addition step".

The amino acid salt mentioned above may be any amino acid salt having a chemical structure such that the hydrogen ion belonging to the carboxyl group in the amino acid has been completely substituted by a cation such as a metal ion and an ammonium ion, without any particular restriction. When the amino acid has a plurality of carboxyl groups, it is preferred that the hydrogen ion at each of the plurality of carboxyl groups has totally been substituted by a sodium ion. Further the amino acid salt may contain a salt species wherein the hydrogen ions at the plurality of carboxyl groups have been only partly substituted by a cation such as a metal ion and an ammonium ion.

The amino acid salt to be used is the L-aspartic acid salt.

The process for producing N-bishydroxyalkyl-amino acids according to the invention may comprise the step of preparing the above amino acid salt.

The step of preparing the above amino acid salt is not particularly restricted. For example, a procedure applicable in substituting the carboxyl hydrogen atom or atoms in the amino acid mentioned above can adequately be applied. In the step of preparing the above amino acid salt, it is preferred that the hydrogen ion at each carboxyl group of the amino acid be totally substituted.

In the above alkylene oxide addition step, the mole ratio between the amino acid salt and alkylene oxide is not particularly restricted but, for example, the ratio (number of moles of alkylene oxide) / (number of moles of amino acid salt) is preferably selected within the range of 2.0 to 4.5, more preferably 2.5 to 4.0, so that the yield of those N-bishydroxyalkyl-amino acids in which the two hydrogens of the amino group of the amino acid salt have totally been substituted by an alkylene oxide may be increased.

The reaction medium shall contain water. Thus, for example, water and mixed media composed of water and a water-soluble organic solvent such as an alcohol may be used. Among them, water is preferably used.

The reaction conditions, the percent conversion of the amino acid salt and other parameters in the above alkylene oxide addition step may be the same, for example, as those in the ethylene oxide addition step mentioned earlier.

In the above alkylene oxide addition step , those N-bishydroxyalkyl-amino acids resulting from complete substitution of the two hydrogen atoms of the amino group in the amino acid salt by an alkylene oxide are generally formed. However, those N-bishydroxyalkyl-amino acids resulting from substitution of one hydrogen atom of the amino group in the amino acid salt by an alkylene oxide may also be formed.

The process for producing N-bishydroxyalkyl-amino acids according to the present invention may comprise, in addition to the above steps, the step of converting the cation at the carboxyl group or groups in the N-bishydroxyalkyl-amino acid produced in the above alkylene oxide addition step to another cation or the hydrogen ion.

The process for producing N-bishydroxyalkyl-amino acids according to the present invention comprises simplified production steps and therefor is advantageous from the production cost viewpoint. Further, the possibility of byproduct formation is low. Furthermore, since the reaction of the alkylene oxide is carried out in an aqueous solution, it is possible to submit to the reaction of an excessive number of moles of the alkylene oxide relative to the number of moles of the amino acid salt. Thus, the production steps are simplified, the possibility of byproduct formation is reduced and, as a result of synergism of these effects, the N-bishydroxyalkyl-amino acids can be produced efficiently in increased yields.

The thus obtained N-bishydroxyalkyl-amino acid comprises a structure wherein at least one carboxyl or substituted carboxyl group and a nitrogen atom are simultaneously present in one and the same molecule; therefore these acids may become at least bidendate against metal ions. As a result, they exhibit good chelating activity.

The preferred N-bishydroxy-alkyl-amino acid is the N-bishydroxyethyl-L-aspartic acid. The process for producing N-bishydroxyethyl-L-aspartic acid comprises the step of effecting an addition reaction of ethylene oxide to L-aspartic acid salt in an aqueous solution. The production steps are simplified, the possibility of byproduct formation is reduced, and N-bishydroxyethyl-L-aspartic acid may be produced in improved yields. Such N-bishydroxyethyl-L-aspartic acid production process is a further aspect of the present invention.

The above step of effecting an adoption reaction of ethylene oxide to L-aspartic acid salt in an aqueous solution is not particularly restricted but may be carried out according to the above-mentioned N-bishydroxyalkyl-amino acid production process using L-aspartic acid salt as the amino acid salt and ethylene oxide as the alkylene oxide.

In the above stated step of effecting an addition reaction of ethylene oxide to L-aspartic acid salt in an aqueous solution, the reaction procedure may be carried out in the same manner as in the above-mentioned N-bishydroxyalkyl-amino acid production process with respect to the reactant ratio, reaction conditions, amino acid salt conversion rate, byproduct, and further steps.

In the N-bishydroxyethylaspartic acid production process of the present invention as well, the same effects can be obtained as in the above-mentioned N-bishydroxyalkyl-amino acid production process.

The thus obtained N-bishydroxyethyl-L-aspartic acid comprises a structure wherein two carboxyl or substituted carboxyl groups and a nitrogen atom simultaneously present in one and the same molecule, and thus may become a tri- or polydentate ligand against metal ions. Accordingly, they exhibit good chelating activity.

The above stated N-bishydroxyethyl-L-aspartic acid, comprises excellent chelating activity and high biodegradability.

The above stated N-bishydroxyethyl-L-aspartic acid is produced by a process comprising the step of effecting an addition reaction of ethylene oxide to L-aspartic acid salt, which is in L form, in an aqueous solution. Thus, the obtained N-bishydroxyethyl-L-aspartic acid is represented by the following general formula (15): 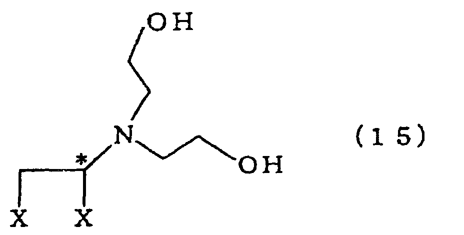 wherein X has the above stated meaning and the asterix * indicates that the asymmetric carbon atom marked with this is in S configuration.

In this case, the N-bishydroxyethyl-L-aspartic acid comprises a chemical structure, wherein an asymmetric carbon atom in S configuration is bonded to a nitrogen atom having ethyl hydroxy groups, so that they show excellent biodegradability.

The above stated N-bishydroxyethyl-L-aspartic acid production process is further useful as a process for producing intermediates for the production of compounds having the basic skeleton of L-aspartic acid within the molecule, for example the above-mentioned amino acid derivative composition, or as a step preceding to the above-mentioned amino acid derivative production process.

In this case, when the above stated N-bishydroxyethyl-L-aspartic acids of general formula (15) is used as an intermediate, the final products, namely compounds having the basic skeleton of L-aspartic acid within the molecule, are produced at a reduced cost, retain the S configuration of the asymmetric carbon atom, and have good biodegradability, for the same reasons as mentioned above.

The N-bishydroxyalkyl-amino acids according to the present invention and the compounds produced by using the same as intermediates are useful as chelating agents. Chelating agents comprising any of these compounds alone or, if necessary, together with another or other compounds having chelating activity and/or an additive or additives can be utilized in a wide range of fields, such as detergents, detergent compositions, textile auxiliaries, auxiliaries in paper and pulp manufacture, agents for metal surface treatment, photographic developers and the like.

### BEST MODE FOR CARRYING OUT THE INVENTION

The following examples illustrate the present invention in further detail. They are, however, not to be construed as to limitate the scope of the invention.

### Production Example 1:

### Ethylene oxide addition step: Production of disodium N,N-bishydroxyethyl-L-aspartate

L-Aspartic acid (26.62 g, 0.2 mole) was added to 40 g of water and, after addition of 16.0 g of sodium hydroxide, water was added until a total weight of 88.56 g was obtained. This aqueous solution was charged in an autoclave and, after sufficient purging with nitrogen and heating to 40 °C, 35.24 g (0.8 mole) of ethylene oxide was added during 3 hours. After completion of the addition, the system was further stirred for 1 hour at 40 °C and the ethylene oxide remaining in the system was removed, whereby 107.69 g of an aqueous solution was obtained. This reaction solution was analyzed by high-performance liquid chromatography, and the conversion of the starting material L-aspartic acid was found to be 100% and the content of disodium N,N-bishydroxyethyl-L-aspartate to be 53.02 g.

### Production Example 2:

### Maleic acid addition step: Production of an L-aspartic acid derivative composition

Maleic anhydride (29.42 g, 0.3 mole), 24.0 g of sodium hydroxide and 4.89 g of lanthanum oxide were added to 53.85 g of the aqueous solution obtained in Production Example 1 and, after pH adjustment to 10.5, the mixture was concentrated to a total weight of 114.65 g. This solution was stirred at 90 °C for 8 hours, then 6.36 g of sodium carbonate was added, and the mixture was further stirred at 80 °C for 1 hour and then allowed to cool to room temperature. The resulting solid precipitate was filtered off and 114.82 g of an aqueous solution was obtained. Analysis of this aqueous solution revealed that the content of the desired L-aspartic acid derivative (A) was 28.89% by weight and that of the L-aspartic acid derivative (B) was 48.93% by weight.

### Production Example 3:

A 50-g portion of the reaction solution obtained in Production Example 2 was treated with DIAION PK216 (trademark; product of Mitsubishi Chemical), a strongly acidic cation exchange resin, and then subjected to a separation procedure using a column packed with DIAION PA316 (trademark; formic acid anion type; product of Mitsubishi Chemical), a strongly basic anion exchange resin, and aqueous solutions of formic acid (0 M to 2.5 M) as eluents, to give 6.86 g of the L-aspartic acid derivative (A) and 12.34 g of the L-aspartic acid derivative (B), each in the acid form.

### Examples 1 and 2 and Comparative Example 1:

### Ca²⁺ chelating ability evaluation

Using the L-aspartic acid derivative (A) [Example 1] and L-aspartic acid derivative (B) [Example 2] obtained in Production Example 3 and [S,S]-ethylenediamine-N,N'-disuccinic acid (ss-EDDS) [Comparative Example 1] as the samples, Ca²⁺ chelating capability evaluation was performed as mentioned above. The results are shown in Table 1. All the samples were used in the Na salt form and the capability was expressed in terms of weight of CaCO₃ trapped per gram of sample (mg CaCO₃/g).

**[Table 1]**

| | | CaCO₃ trapped per g sample (Na salt) |
|---|---|---|
| Ex. 1 | L-Aspartic acid deriv. (A) | 200 mg CaCO₃/g |
| Ex. 2 | L-Aspartic acid deriv. (B) | 280 mg CaCO₃/g |
| Compar.Ex.1 | ss-EDDS (4Na) | 160 mg CaCO₃/g |

In Table 1, ss-EDDS (4Na) denotes the Na salt form of ss-EDDS.

### Example 3 and Comparative Examples 2 to 5:

### H₂O₂ stabilization test

Using the L-aspartic acid derivative (B) [Example 3] obtained in Production Example 3, diethylenetriaminepentaacetic acid (DTPA) [Comparative Example 2], ethylenediaminetetraacetic acid (EDTA), [Comparative Example 3], nitrilotriacetic acid (NTA) [Comparative Example 4] and a blank (no chelating agent) [Comparative Example 5] as the samples, the H₂O₂ stabilization test mentioned above was carried out. The results are shown in Table 2 and Fig. 1. All the samples were used in the Na salt form.

**[Table 2]**

| Time (min.) | Example 3 | Compar. Ex. 2 | Compar. Ex. 3 | Compar. Ex. 4 | Compar. Ex. 5 |
|---|---|---|---|---|---|
| | L-Aspartic acid deriv. (B) | DTPA (5Na) | EDTA (4Na) | NTA (3Na) | Blank (no chelating agent) |
| 0 | 100 | 100 | 100 | 100 | 100 |
| 30 | 85.5 | 82.1 | 80.7 | 14.4 | 0 |
| 60 | 84.6 | 80.1 | 77.1 | 7.5 | 0 |

In Table 2, DTPA (5Na), EDTA (4Na) and NTA (3Na) denotes the Na salt of DTPA, of EDTA and of NTA, respectively.

### Examples 4 and 5:

### Biodegradability testing

The L-aspartic acid derivative (B) [Example 4] obtained in Production Example 3 and the optically inactive counterpart [Example 5], which has the same structure as the above derivative (B), as the samples, a biodegradability test was carried out according to the above-mentioned test method 301C: Modified MITI test (I). The results are shown in Table 3

**[Table 3]**

| | Percent decomposition after 28 days based on residual DOC (%) |
|---|---|
| Example 4 | 35 |
| Example 5 | 10 |

In Table 3, each numerical value indicates the percent decomposition after 28 days based on the residual DOC.

### Production Example 4:

L-Aspartic acid (26.62 g, 0.2 mole) was added to 40 g of water and, after addition of 16.0 g of sodium hydroxide, water was added until a total weight of 88.56 g was obtained. This aqueous solution was charged in an autoclave and, after sufficient purging with nitrogen and heating to 40 °C, 35.24 g (0.8 mole) of ethylene oxide was added over 3 hours. After completion of the addition, the system was further stirred for 1 hour at 40 °C and the ethylene oxide remaining in the system was removed, whereby 107.69 g of an aqueous solution was obtained. This reaction solution was analyzed by high-performance liquid chromatography, and the conversion of the starting material L-aspartic acid was found to be 100% and the content of disodium N,N-bishydroxyethyl-L-aspartate to be 53.02 g.

## Claims

1. An L-aspartic acid derivative represented by the following general formula (2): 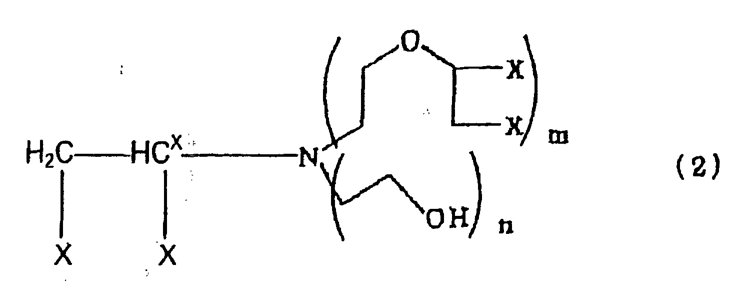 wherein:
C^{x} indicates an asymmetric carbon atom being in the S configuration;
X represents COOM, wherein all the M are the same or different and each represents a hydrogen atom, an alkali metal atom, an alkaline earth metal atom or an ammonium group;
m represents an integer of 1 or 2; and
n represents an integer of 0 or 1 with the further proviso that m + n is equal to 2.

2. An L-aspartic acid derivative composition
which comprises the L-aspartic acid derivative according to claim 1,
wherein said L-aspartic acid derivative composition comprises an L-aspartic acid derivative (A) and an L-aspartic acid derivative (B),
wherein said L-aspartic acid derivative (A) being represented by the following general formula (3): 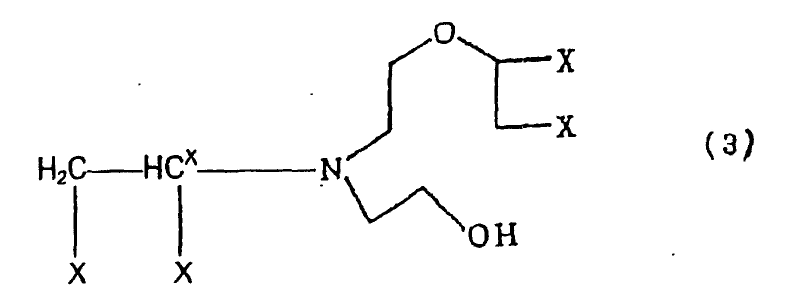 wherein:
C^{x} Indicates an asymmetric carbon atom being in the S configuration;
X represents COOM, wherein all the M are the same or different and each represents a hydrogen atom, an alkali metal atom, an alkaline earth metal atom or an ammonium group; and
wherein said L-aspartic acid derivative (B) being represented by the following general formula (4): 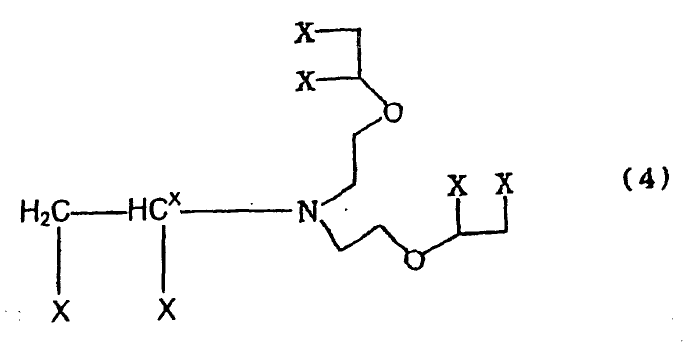 wherein:
C^{x} indicates an asymmetric carbon atom in the S configuration;
X represents COOM, wherein all the M are the same or different and each represents a hydrogen atom, an alkali metal atom, an alkaline earth metal atom or an ammonium group; and
wherein said L-aspartic acid derivative composition comprises a mole ratio of said L-aspartic acid derivative (A) to said L-aspartic acid derivative (B) in the range of from 1 : 99 to 99 : 1.

3. A process for producing the L-aspartic acid derivative according to claim 1, which comprises the steps of:
- subjecting L-aspartic acid to an addition reaction with ethylene oxide in order to obtain a L-aspartic acid/ethylene oxide adduct; and
- reacting said L-aspartic acid-ethylene oxide adduct with maleic acid in the presence of a rare earth-based catalyst.

4. A process for producing the L-aspartic acid derivative composition according to claim 2,
which comprises the steps of:
- subjecting L-aspartic acid to an addition reaction with ethylene oxide in order to obtain a L-aspartic acid/ethylene oxide adduct; and
- reacting said L-aspartic acid-ethylene oxide adduct with maleic acid in the presence of a rare earth-based catalyst.

5. A process for producing N-bishydroxyalkyl-amino acid derivatives represented by the following general formula (5): 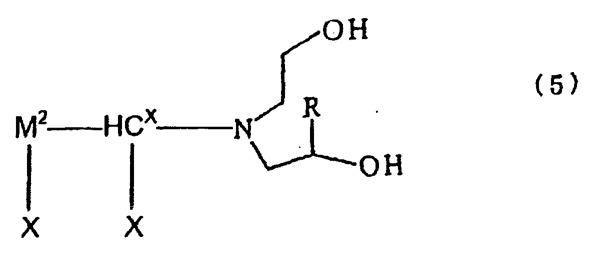 wherein:
C^{x} indicates an asymmetric carbon atom in the S configuration;
R represents a hydrogen atom;
M² represents -CH₂- group;
X represents COOM, wherein all the M are the same or different and each represents a hydrogen atom, an alkali metal atom, an alkaline earth metal atom or an ammonium group;
which comprises the step of effecting an addition reaction of an alkylene oxide to an amino acid salt in an aqueous solution.

6. Use of the L-aspartic acid deivative according to claim 1 as a chelating agent.

7. Use of the L-aspartic acid derivative composition according to claim 2 as a chelating agent.

## Patentansprüche

1. L-Asparaginsäure-Derivat
entsprechend der nachstehenden allgemeinen Formel (2): 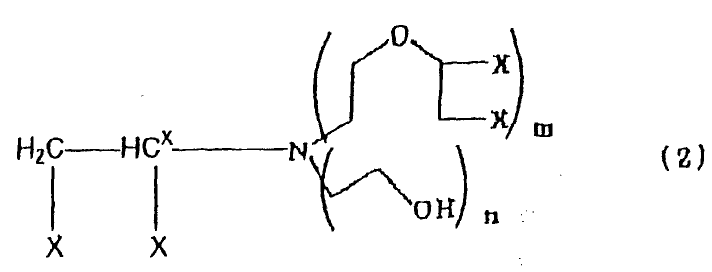 wobei:
C^{x} steht für ein asymmetrisches Kohlenstoffatom in S-Konfiguration;
X steht für die Gruppe COOM, wobei alle Elemente M gleich oder verschieden sind und je stehen für ein Wasserstoffatom, für ein Alkalimetallatom, für ein Erdalkalimetallatom oder für eine Ammoniumgruppe;
"m" steht für eine ganze Zahl, nämlich 1 oder 2; und
"n" steht für eine ganze Zahl, nämlich 0 oder 1, mit der weiteren Maßgabe, dass m + n gleich 2 ist.

2. L-Asparaginsäure-Derivatzusammensetzung,
die das L-Asparaginsäure-Derivat nach Anspruch 1 aufweist,
wobei diese L-Asparaginsäure-Derivatzusammensetzung ein
L-Asparaginsäure-Derivat (A) und ein L-Asparaginsäure-Derivat (B) enthält,
wobei dieses L-Asparaginsäure-Derivat (A) der nachstehenden allgemeinen Formel (3) entspricht: 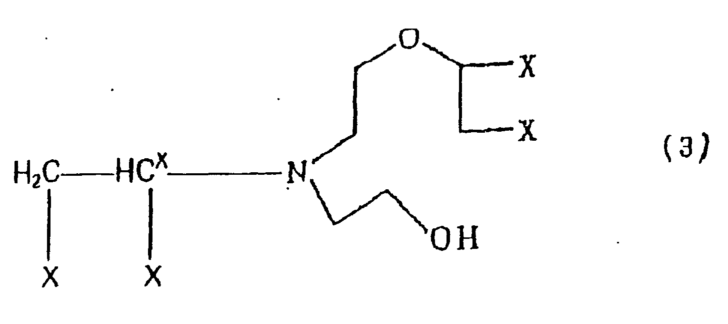 wobei:
C^{x} steht für ein asymmetrisches Kohlenstoffatom in S-Konfiguration;
X steht für die Gruppe COOM, wobei alle Elemente M gleich oder verschieden sind und je stehen für ein Wasserstoffatom, für ein Alkalimetallatom, für ein Erdalkalimetallatom oder für eine Ammoniumgruppe; und
wobei dieses L-Asparaginsäure-Derivat (B) der nachstehend allgemeinen Formel (4) entspricht: 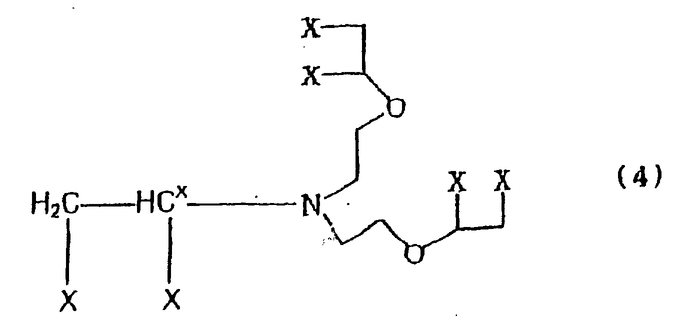 wobei:
C^{x} steht für ein asymmetrisches Kohlenstoffatom in S-Konfiguration;
X steht für die Gruppe COOM, wobei alle Elemente M gleich oder verschieden sind und je stehen für ein Wasserstoffatom, für ein Alkalimetallatom, für ein Erdalkalimetallatom oder für eine Ammoniumgruppe; und
wobei diese L-Asparaginsäure-Derivatzusammensetzung ein Molverhältnis von diesem L-Asparaginsäure-Derivat (A) zu diesem L-Asparaginsäue-Derivat (B) im Bereich von 1 : 99 zu 99 : 1 aufweist.

3. Verfahren zur Herstellung des L-Asparaginsäure-Derivat nach Anspruch 1, **gekennzeichnet durch** die Verfahrensschritte:
- L-Asparaginsäure wird einer Ethylenoxid-Additionsreaktion unterworfen, um ein L-Asparaginsäure/Ethylenoxid-Additionsprodukt zu erzeugen; und
- dieses L-Asparaginsäure/Ethylenoxid-Additionsprodukt wird mit Maleinsäure in Gegenwart eines Seltenerdmetall-Katalysators umgesetzt.

4. Verfahren zur Herstellung der L-Asparaginsäure-Derivalzusammensetzung nach Anspruch 2,
**gekennzeichnet durch** die Verfahrensschritte:
- L-Asparaginsäure wird einer Ethylenoxid-Additionsreaktion unterworfen, um ein L-Asparaginsäure/Ethylenoxid-Additionsprodukt zu erzeugen; und
- dieses L-Asparaginsäure/Ethylenoxid-Additionsprodukt wird mit Maleinsäure in Gegenwart eines Seltenerdmetall-Katalysators umgesetzt.

5. Verfahren zur Herstellung von N-Bishydroxyalkyl-aminosäure-Derivaten entsprechend der nachstehenden allgemeinen Formel (5): 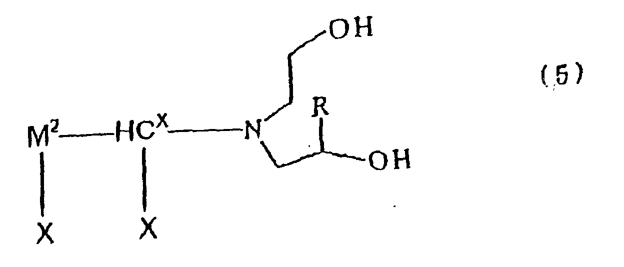 wobei:
C^{x} steht für ein asymmetrisches Kohlenstoffatom in S-Konfiguration;
R steht für ein Wasserstoffatom;
M² steht für eine -CH₂-Gruppe;
X steht für die Gruppe COOM, wobei alle Elemente M gleich oder verschieden sind und je stehen für ein Wasserstoffatom, für ein
Alkalimetallatom, für ein Erdalkalimetallatom oder für eine Ammoniumgruppe;
**gekennzeichnet durch** den Verfahrensschritt:
- ein Aminosäure-Salz wird in wässriger Lösung einer Alkylenoxid-Additionsreaktion unterworfen.

6. Verwendung
des L-Asparaginsäure-Derivat nach Anspruch 1
als Chelatbildner.

7. Verwendung
der L-Asparaginsäure-Derivatzusammensetzung nach Anspruch 2
als Chelatbildner.

## Revendications

1. Un dérivé d'acide L-aspartique
conformément à la formule générale (2) suivante 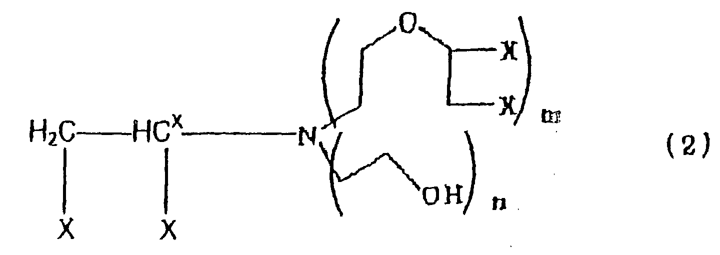 dans laquelle
C^{x} représente un atome de carbone asymétrique ayant la configuration S ;
X représente le groupe COOM, dans lequel tous les éléments M sont identiques ou non et dans lequel chacun représente un atome d'hydrogène, un atome de métal alcalin, un atome de métal alcalino-terreux ou un groupe ammonium ;
m étant un nombre entier, notamment 1 ou 2 ; et
n étant un nombre entier, notamment 0 ou 1, avec la condition supplémentaire que la somme de (m+n) est égale 2.

2. Une composition du dérivé d'acide L-aspartique
qui comprend le dérivé d'acide L-aspartique conformément à la revendication 1,
cette composition du dérivé d'acide L-aspartique contenant un dérivé d'acide L-aspartique (A) et un dérivé d'acide L-aspartique (B),
dans laquelle ce dérivé d'acide L-aspartique (A) correspond à la formule générale (3) suivante : 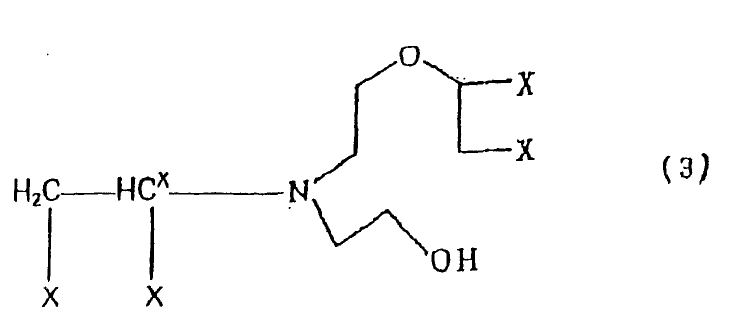 dans laquelle
C^{x} représente un atome de carbone asymétrique ayant la configuration S ; X représente le groupe COOM, dans lequel tous les éléments M sont identiques ou non et dans lequel chacun représente un atome d'hydrogène, un atome de métal alcalin, un atome de métal alcalino-terreux ou un groupe ammonium ; et
dans laquelle
le dérivé d'acide L-aspartique (B) correspond à la formule générale (4) suivante : 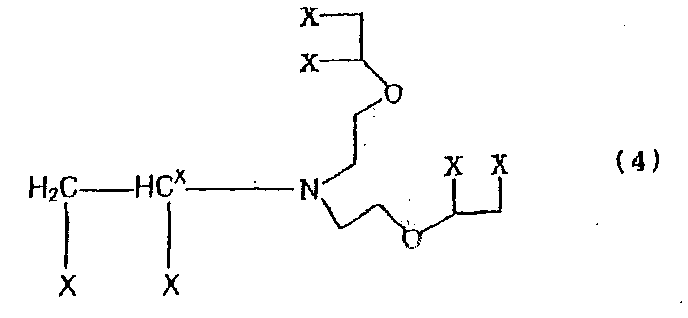 dans laquelle
C^{x} représente un atome de carbone asymétrique ayant la configuration S ;
X représente le groupe COOM, dans lequel tous les éléments M sont identiques ou non et dans lequel chacun représente un atome d'hydrogène, un atome de métal alcalin, un atome de métal alcalino-terreux ou un groupe ammonium ; et dans laquelle
cette composition du dérivé d'acide L-aspartique présente un rapport molaire dérivé d'acide L-aspartique (A) / dérivé d'acide L-aspartique (B) compris dans la plage entre 1 : 99 et 99 : 1.

3. Procédé de fabrication du dérivé d'acide L-aspartique conformément à la revendication 1,
**caractérisé par** les étapes de fabrication suivantes :
- soumettre l'acide L-aspartique à une réaction d'addition avec l'oxyde d'éthylène pour obtenir un adduit acide L-aspartique / oxyde d'éthylène; et
- faire réagir cet adduit acide L-aspartique / oxyde d'éthylène avec l'acide maléique en présence d'un catalyseur à base de terres rares.

4. Procédé de fabrication de la composition du dérivé d'acide L-aspartique conformément à la revendication 2,
**caractérisé par** les étapes de fabrication suivantes :
- soumettre l'acide L-aspartique à une réaction d'addition avec l'oxyde d'éthylène pour obtenir un adduit acide L-aspartique / oxyde d'éthylène; et
- faire réagir cet adduit acide L-aspartique / oxyde d'éthylène avec l'acide maléique en présence d'un catalyseur à base de terres rares.

5. Procédé de fabrication de dérivés d'amino-acide N-bis(hydroxy-alkyl) qui correspond à la formule générale (5) suivante : 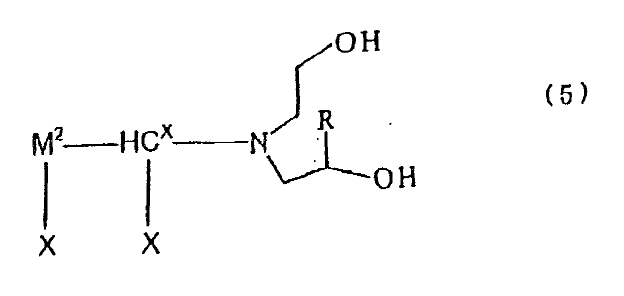 dans laquelle
C^{x} représente un atome de carbone asymétrique ayant la configuration S ;
R représente un atome d'hydrogène ;
M² représente un groupe -CH₂ ;
X représente le groupe COOM, dans lequel tous les éléments M sont identiques ou non et dans lequel chacun représente un atome d'hydrogène, un atome de métal alcalin, un atome de métal alcalino-terreux ou un groupe ammonium ; et qui est
**caractérisé par** l'étape de fabrication suivante :
- soumettre le sel d'amino-acide dans une solution aqueuse à une réaction d'addition avec l'oxyde d'alkylène.

6. Emploi
du dérivé d'acide L-aspartique conformément à la revendication 1, en tant qu'agent chélateur.

7. Emploi
de la composition du dérivé d'acide L-aspartique conformément à la revendication 2,
en tant qu'agent chélateur.
